# EUROPÄISCHE PATENTSCHRIFT

(11) **EP 1 893 770 B1**
(45) Veröffentlichungstag und Bekanntmachung des Hinweises auf die Patenterteilung: **14.03.2012**
(21) Anmeldenummer: 06755095.4
(22) Anmeldetag: 09.05.2006
(51) Int. Cl.: C12Q 1/00, C12Q 1/68, B01L 3/00, G01N 33/543

(54) **VORRICHTUNG UND VERFAHREN ZUR NORMALISIERUNG VON NUKLEINSÄURE-KONZENTRATIONEN**
DEVICE AND METHOD FOR STANDARDIZING NUCLEIC ACID CONCENTRATIONS
DISPOSITIF ET PROCEDE DE NORMALISATION DE CONCENTRATIONS D'ACIDES NUCLEIQUES

(30) Priorität: 30.05.2005 DE 102005025080
(43) Veröffentlichungstag der Anmeldung: 05.03.2008
(73) Patentinhaber: Qiagen GmbH, 40724 Hilden (DE)
(72) Erfinder: HIMMELREICH, Ralf, 40764 Langenfeld (DE); ERBACHER, Christoph, 42781 Haan (DE); LÖFFERT, Dirk, 40589 Düsseldorf (DE)
(86) Internationale Anmeldenummer: PCT/EP2006/062153
(87) Internationale Veröffentlichungsnummer: WO 2006/128776

(56) Entgegenhaltungen:
- EP-A- 1 324 042
- WO-A-00/29112
- WO-A-2005/047545
- WO-A2-03/091304
- US-A1- 2004 053 256
- US-A1- 2004 209 269
- US-A1- 2004 265 476
- US-A1- 2005 064 469
- US-B1- 6 485 915

## Beschreibung

Die vorliegende Erfindung betrifft ein Verfahren und ein Kit zur durchführen des Verfahrens zur Normalisierung von Nukleinsäure-Konzentrationen, bevorzugt zur Normalisierung von Nukleinsäure-Konzentrationen bei enzymatischen Nukleinsäure-Amplifikations-und Modifikations-Verfahren.

Ein bekanntes Problem in dem die Erfindung betreffenden technischen Gebiet ist, dass Nukleinsäure-Präparationen in Abhängigkeit vom Ausgangsmaterial und/oder dem Verfahren der Aufreinigung Schwankungen in der finalen Konzentration an gereinigter Nukleinsäure zeigen. Daher wird bei Nukleinsäure-Amplifikations- und Modifikations-Verfahren in der Regel die gereinigte Nukleinsäure vor der Reaktion quantifiziert und gegebenenfalls auf eine standardisierte Konzentration eingestellt, das heißt, die Proben werden normalisiert. Nur dann ist eine sinnvolle Quantifizierung und Vergleich der Proben mittels quantitativer PCR oder anderer Nachweisverfahren möglich. Eine weitere Möglichkeit der Quantifizierung besteht in der simultanen Amplifikation eines Standards, Diese Standards stellen definierte Nukleinsäure-Sequenzen und dienen als Marker. Der Sequenzursprung kann sogar aus einem anderen Organismus stammen. Die Aufgabe dieser Standards ist es, die Reaktionsbedingungen anzuzeigen und eine Analyse der Daten zu ermöglichen.

Aus dem Stand der Technik sind alternative Präparationsverfahren bekannt, (beispielsweise das 'IQ System' von Promega, Madison, USA oder 'Charge Switch gDNA Normalized Buccal Cell Kit' von Invitrogen, Carlsbad, USA), die eine Präparation von einheitlichen Konzentrationen an Nukleinsäure (vornehmlich genomische DNA) aus unterschiedlichen Ausgangsmaterialien erlauben sollen. Es hat sich jedoch in der Praxis gezeigt, dass diese Technologien immer noch in den Ausbeuten eine extrem starke Schwankung aufweisen (Standardabweichung vom Mittelwert >>10%), eine einheitliche Konzentration an Nukleinsäuren ist somit nicht gegeben.

Derzeit ist aus dem Stand der Technik kein Präparationsverfahren bekannt, für welches eine Quantifizierung und Normalisierung nicht erforderlich ist, sofern die vorgesehene Downstream-Reaktion eine solche voraussetzt. Für ein Verfahren, welches eine einfache und genaue Normalisierung ermöglicht, besteht somit ein starker Bedarf in der Fachwelt.

Die aus dem Stand der Technik bekannten Nachteile werden durch die vorliegende Erfindung gelöst. Diese ermöglicht eine effektive, präzise, einfache und schnelle Normalisierung der gereinigten Nukleinsäure ohne Quantifizierung. Dies wird erreicht mittels modifizierter Verbrauchsmaterialien.

Die vorliegende Erfindung besteht aus einem Verfahren zur Normalisierung von Nukleinsäure-Konzentrationen und einem Kit nach den Ansprüchen 1 bis 4.

Vorrichtungen aufweisend eine modifizierte Oberfläche mit definierter Nukleinsäure-Bindekapazität, können beispielsweise PCR-Gefäße (z.B. Eppendorf Tubes), Multiwell-Platten (z.B. 96-well-Platten oder 384-well-Platten), aber auch Folien oder so genannte 'Dip-Sticks' sein. Unter 'Dip-Sticks' im Sinne der Erfindung werden vorzugsweise Stäbe aus Glas oder Kunststoff mit einer erfindungsgemäß modifizierten Oberfläche verstanden, die in eine Nukleinsäurehaltige Lösung eingetaucht werden und die Nukleinsäuren an die Oberfläche binden können. Formen, Materialien und Oberflächengestaltung solcher Dip-Sticks sind dem Fachmann hinlänglich bekannt. Grundsätzlich kann die gesamte mit der Nukleinsäure-haltigen Lösung in Kontakt stehende Oberfläche (beispielsweise das gesamte Innere eines PCR-Tubes) modifiziert sein, oder aber definierte Bereiche der Oberfläche können modifiziert sein.

Die Bindekapazität der modifizierten Oberfläche für Nukleinsäuren beruht auf einer chemischen Funktionalisierung, welche eine reversible Bindung der Nukleinsäuren zulässt. Die Bindung der Nukleinsäuren kann beispielsweise über an der Oberfläche immobilisierte Nukleinsäuren oder Nukleinsäureanaloga erfolgen. Diese liegen vorzugsweise in Form von Oligonukleotiden vor. Unter immobilisierten Nukleinsäuren oder Nukleinsäureanaloga im Sinne der Erfindung wird DNA, RNA, DNA-RNA-Hybride, PNA und locked nucleic acids verstanden. Weitere Nukleinsäureanaloga, die eine reversible Bindung mit Nukleinsäuren eingehen können, sind dem Fachmann wohlbekannt und können in der Erfindung ebenfalls zum Einsatz kommen. Die Sequenz der immoblisierten Nukleinsäuren oder Nukleinsäureanaloga kann zufällig (random) sein und somit eine unspezifische Bindung der Nukleinsäuren bedingen, sie kann aber auch eine OligoT-Sequenz zur spezifischen Bindung von eukaryotischer mRNA am PolyA-Schwanz aufweisen oder aber eine definierte Sequenz zur sequenzspezifischen Bindung von Nukleinsäuren aufweisen. Die Sequenz kann auch so gewählt werden, dass sich mit zu bindender doppelsträngiger Nukleinsäure eine triple helix bildet.

Die Bindekapazität der modifizierten Oberfläche für Nukleinsäuren kann weiterhin auf ionischen Schichten beruhen, beispielsweise Beschichtungen mit Anionen- oder Kationentauscher-Material. Typische Anionenaustauscher-Materialien sind in der Fachwelt hinlänglich bekannt und ermöglichen unter spezifischen Bedingungen die reversible und unspezifische Bindung von Nukleinsäuren an die Oberfläche. Die Bindung der Nukleinsäuren ist reversibel und kann beispielsweise durch Hitze, z.B. durch Erhitzen der Probe in einer PCR, gelöst werden. Typische Kationentauscher-Materialien weisen beispielsweise Oberflächen auf, die Sulfonat-, Carboxyl- und/oder Phosphat-Gruppen auf der Oberfläche tragen. Die reversible Bindung von Nukleinsäuren an Kationentauscher ist in der Fachwelt hinlänglich bekannt.

Auch hydrophobe Schichten können die Bindekapazität der modifizierten Oberfläche für Nukleinsäuren herstellen. Eine solche Schicht besteht beispielsweise aus Polypropylen. Viele handelsübliche Reaktionsgefäße (z.B. Eppendorf-Tubes) bestehen aus Polypropylen. Um eine definierte hydrophobe Fläche zu schaffen, können z.B. Teile der Oberfläche einer solchen Vorrichtung aus Polypropylen hydrophilisiert werden. Zur reversiblen Bindung von Nukleinsäuren aus einer Lösung an eine hydrophobe Oberfläche müssen diese zunächst mit einem Molekül versetzt werden, welches eine positive Ladung und einen hydrophoben Rest aufweist um eine Interaktion zu ermöglichen, beispielsweise mit Diethylammonium-Ionen. Dem Fachmann sind alternative Verfahren zur reversiblen Bindung von Nukleinsäuren an hydrophobe Oberflächen wohlbekannt.

Die erfindungsgemäßen Vorrichtungen können optional auf ihrer Oberfläche neben der oben beschriebenen ersten modifizierten Oberfläche mit Bindekapazität für Nukleinsäuren auch eine eben solche zweite modifizierte Oberfläche aufweisen. Da die Bindekapazität der ersten modifizierten Oberfläche durch die Ausdehnung der Oberfläche und deren chemische Funktionalisierung limitiert wird, bindet immer eine konstante Menge Nukleinsäure an diese Oberfläche. Überschüssige Nukleinsäure kann aus der Vorrichtung entfernt werden (beispielsweise durch Abschütten der Probe, Spülen der Vorrichtung, etc.). Sollte sich jedoch eine zu geringe Menge Nukleinsäure in der Probe befinden, so dass die Bindekapazität der ersten modifizierten Oberfläche über die zur Verfügung stehende Nukleinsäuremenge hinausgeht, so kann mit der ersten modifizierten Oberfläche keine exakte Normierung der Nukleinsäuremenge für nachfolgende Reaktionen mehr vorgenommen werden. In diesem Fall würden sich beispielsweise in einer quantitativen PCR nur sehr geringe Mengen an Nukleinsäuren nachweisen lassen. Es kann in einem solchen Fall nicht bestimmt werden, ob dieses durch beispielsweise eine schlechte Probenquatität und/oder eine zu geringe Quantität an Ausgangsmaterial und/oder beispielsweise auch an einem fehlerhaft angesetzten Reaktionsgemisch hervorgerufen wird. Ein Kontrolle der Reaktion erfolgt durch die optionale zweite modifizierte Oberfläche, welche ebenfalls eine definierte Bindekapazität für Nukleinsäuren besitzt. An der zweiten modifizierten Oberfläche ist im Gegensatz zur ersten modifizierten Oberfläche jedoch bereits eine definierte Menge Nukleinsäure, das heißt DNA oder RNA, einer definierten Sequenz immobilisiert. Die Sequenz und die Länge dieser Nukleinsäuren wird so bestimmt, dass sie für die nachfolgende Reaktion, beispielsweise eine PCR oder eine RT-PCR geeignet ist. In einer PCR oder RT-PCR wird diese Nukleinsäure über geeignete Primer amplifiziert. Die hier bereits gebundenen Nukleinsäuren dienen in einer nachfolgenden Reaktion als Standard. Somit wird eine exakte Quantifizierung der Nukleinsäure aus der Probe möglich und gleichzeitig dient der Standard zur Kontrolle der Effizienz der nachfolgenden Reaktion. Die Bindung der Nukleinsäuren an diese zweite modifizierte Oberfläche kann beispielsweise kovalent erfolgen, die Amplifikation des Standards erfolgt dann beispielsweise über eine Festphasen-PCR. Die Nukleinsäuren können jedoch auch nicht-kbvalent an die zweite Modifizierte Oberfläche gebunden sein, dürfen sich jedoch von dieser erst bei Beginn der nachfolgenden Reaktion und nicht etwa bei Befüllen der Vorrichtung mit der Probe oder beim Waschen der Vorrichtung von dieser Lösen. Im Sinne der vorliegenden Erfindung können die Nukleinsäuren an eine zweite modifizierte Oberfläche gebunden werden, die eine Anionen- oder Kationentauscher-Oberfläche aufweist, oder eine hydrophobe Oberfläche aufweist oder aber die Bindung der Nukleinsäuren erfolgt über an die Oberfläche gebundene Nukleinsäuren. Die Möglichkeiten sind grundsätzlich gleich zu denen, Nukleinsäuren aus der Probe an die erste modifizierte Oberfläche zu binden (siehe oben). Voraussetzung für die Bindung von Nukleinsäuren an die zweite modifizierte Oberfläche ist weiterhin, dass die zweite modifizierte Oberfläche vollkommen mit der als Standard dienenden Nukleinsäure gesättigt ist, um keine Schwankungen der Bindekapazität für die aus der Probe stammende Nukleinsäure zu verursachen. Als Standard dienenden Nukleinsäuren können in einer alternativen Ausführungsform der Erfindung auch an Oberflächen gebunden sein, die der erfindungsgemäßen Vorrichtung erst vor Beginn der nachfolgenden Reaktion zugefügt werden, beispielsweise mittels Kunststoff oder Glas-Partikels, z.B. in Form von Plättchen oder Kugeln etc., auf die eine definierte Menge als Standard dienender Nukleinsäure aufgebracht ist, z.B. über eines der oben aufgeführten Verfahren. Dem Fachmann sind Größe, Form und Ausführungsart solcher Vorrichtungen vertraut. Alternativ kann eine als Standard dienende Nukleinsäure auch vor der nachfolgenden Reaktion der Probe zupipettiert werden, wobei die ersten beiden genannten Möglichkeiten jedoch eventuelle Ungenauigkeiten des Pipettierens ausschließen.

Die Modifizierung der Oberfläche der Vorrichtung kann, je nach dem, auf welche Weise die Nukleinsäuren an die Vorrichtung gebunden werden sollen, auf unterschiedliche Weise erfolgen. In der Folge sind einige Beispiele für geeignete Verfahren aufgeführt. Es können jedoch jegliche weiteren Verfahren, die dem Fachmann dienlich scheinen, verwendet werden.

Plasma-Verfahren (Niederdruck-, bevorzugt aber Atmosphärendruck-Plasmen): Atmosphärendruck betriebene gepulste Barriereentladungen werden seit langer Zeit für Oberflächenaktivierung von Polymeren für das anschließende Bedrucken, Bekleben oder Lackieren eingesetzt. Ein neues Anwendungsgebiet für diese Entladung eröffnet sich durch ihre Verwendung für plasmagestützte Beschichtungs-und Reinigungsprozesse bei Atmosphärendruck. Durch die Einspeisung von unter den angewandten Bedingungen gasförmigen Verbindungen wie z.B. Glycidylmethacrylat, Acrylsäure, Fluorkohlenwasserstoffe, Silicium-organische Verbindungen, N₂ oder O₂ und andere Verbindungen in den Entladungsbereich, lassen sich auf Substraten Schichten abscheiden oder im Fall von N₂ und O₂ kovalente Modifikationen von z.B. Kunststoffen mit Aminogruppen bzw. Carboxy- und Hydroxylgruppen erreichen. Aufgrund der Möglichkeiten, diese Entladung in sehr kleinen Volumina zu unterhalten, eröffnen sich neue Perspektiven für die strukturierte Modifizierung der chemischen und physikalischen Eigenschaften von Oberflächen sowie für die Behandlung innerer Oberflächen von Vorrichtungen, beispielsweise Mikrofluidik-Bauteilen oder kleinen Probegefäßen, wie z.B. PCR-Gefäße. Das Verfahren kann dazu eingesetzt werden, gezielt und regioselektiv Nukleinsäure-bindende Bereiche in einem PCR-Gefäß zu erzeugen, um damit eine definierte Menge an Nukleinsäuren reversibel zu binden.

### CVD-Verfahren (Chemical Vapor Deposition)

Dieses Verfahren wird oft in Kombination mit Plasma-Verfahren eingesetzt. Die Vorrichtung, beispielsweise ein PCR-Gefäß, wird einem Dampf ausgesetzt der eine oder mehrere zersetzliche oder reaktive chemische Ausgangsverbindungen enthält. Die Zersetzungsreaktion findet an der Oberfläche der Vorrichtung statt und die Zersetzungsprodukte lagern sich ab wodurch die Oberfläche beschichtet wird. Das Verfahren kann dazu eingesetzt werden, gezielt und regioselektiv Nukleinsäure bindende Bereiche in einer Vorrichtung, wie beispielsweise einem PCR-Gefäß, zu erzeugen und eine definierte Menge Nukleinsäure reversibel zu binden.

### PVD-Verfahren (Physical Vapor Deposition)

Dieses Verfahren ist dadurch gekennzeichnet, dass der abgeschiedene Stoff keine chemische Veränderung erfährt und physikalisch so abgeschieden wird, wie er in der Gasphase vorlag. Das Verfahren kann dazu eingesetzt werden gezielt und regioselektiv Nukleinsäure bindende Bereiche in einer Vorrichtung, beispielsweise in einem PCR-Gefäß, zu erzeugen und eine definierte Menge Nukleinsäure reversibel zu binden.

Bekannte nass-chemische Verfahren können ebenfalls dazu eingesetzt werden Nukleinsäure-bindende Oberflächen in einer Vorrichtung wie etwa einem PCR-Gefäß zu erzeugen, z.B. durch Oxidation der Oberfläche des PCR-Gefäßes mittels stark oxidierender Säuren, sodass Carboxylgruppen, Aldehydgruppen und/oder Hydroxylgruppen gebildet werden, um nach weiteren nass-chemischen Verfahrensschritten Nukleinsäure-bindende Bereiche in der Vorrichtung zu erhalten und eine definierte Menge Nukleinsäure reversibel zu binden. Auch andere nass-chemische Verfahren können eingesetzt werden, beispielsweise solche, bei denen Affinitätsliganden in einer Plasma-aktivierten oder chemisch funktionalisierten Vorrichtung, etwa ein PCR-Gefäß, kovalent zu immobilisieren um damit Nukleinsäuren zu binden. Beispielsweise können Carbodiimide zur Kopplung der Affinitätsliganden eingesetzt werden. Beispielsweise kann EDC (1-Ethyl-3-(3-dimethylaminopropyl)carbodiimid-Hydrochlorid) eingesetzt werden um eine carboxylierte Oberfläche über eine so genannte Reaktivester-Zwischenstufe zu aktivieren. Der an der Oberfläche gebildete Ester, z.B. Hydroxybenzotriazolester oder N-Hydroxysuccinimidester, wird dann in einem zweiten Schritt mit einem aminofunktionalisierten Affinitätsliganden umgesetzt. Als Affinitätsliganden kommen Biotin, Streptavidin, aminofunktionalisierte Oligonukleotide, PNA, Nukleinsäure bindende Proteine, Antikörper oder ähnliche, dem Fachmann hilfreich erscheinende Affinitätsliganden in Betracht.

Nukleinsäure-bindende Kunststoffoberflächen können auch dadurch erzeugt werden, dass Nukleinsäure-bindende Additive in einem Spritzgussverfahren zur Herstellung der Vorrichtung, beispielsweise eines PCR-Gefäßes, eingesetzt werden. Bevorzugt werden dabei Polymeradditive, die ionische Gruppen wie Ammonium- oder Phosphonium-Gruppen, Caboxyl-Gruppen, Sulfonat- oder Phosphat-Reste enthalten. Diese Polymeradditive führen zu einer Änderung der Oberflächenladung der spritzgegossenen Vorrichtung. Unter geeigneten Bindebedingungen können dann reversibel Nukleinsäuren an der Oberfläche der erfindungsgemäßen Vorrichtung immobilisiert werden. So können bereits bei der Herstellung der Vorrichtung die gewünschten Nukleinsäure-bindenden Eigenschaften erhalten werden. Es ist auch möglich, ausgehend von dieser Nukleinsäure-bindenden Oberfläche mittels regioselektiver Plasmamaskentechnik Muster in der Oberfläche der Vorrichtung zu erzeugen, sodass Nukleinsäure-bindende und -nicht-bindende Bereiche erhalten werden können.

Verfahren der Pfropfpolymerisation sind ebenfalls geeignet, die Oberfläche einer erfindungsgemäßen Vorrichtung zu modifizieren. Bei diesen Verfahren wird durch radikalische Polymerisation ein dünner Polymerfilm hergestellt, der kovalent mit der Gefäßoberfläche verbunden ist. Die radikalische Polymerisation kann dabei photoinitiiert sein aber auch thermisch oder durch energiereiche Strahlung initiiert werden. In der Vorrichtung werden geeignete Monomere vorgelegt, die entweder über ionische Gruppen verfügen oder über reaktive Gruppen, wie z.B. Epoxygruppen, zur Anbindung weiterer chemischer Funktionalitäten, die geeignet sind, Nukleinsäure reversibel zu binden.

Beschichtungen der erfindungsgemäßen Vorrichtung, die zur Ausbildung nichtkovalenter Bindungen befähigt sind, mittels "Dip-coating" mit einem Nukleinsäure-bindenden Polymer sind ebenfalls geeignet. Bevorzugte Polymere sind in diesem Zusammenhang ionische Polymere mit Ammonium-, Phosphonium-, Sulfonium-, Carboxy-, Sulfonat- oder Phosphat-Gruppen. Das Polymer kann dabei auch eine oder mehr der genannten funktionellen Gruppen aufweisen. Bei diesem Verfahren wird die Vorrichtung mit dem zur Beschichtung bestimmten Polymer, welches in einer bestimmten Konzentration in einem Lösungsmittel gelöst vorliegt, in Kontakt gebracht. Nach dem Entfernen des Lösungsmittels, wie etwa Wasser oder organische Lösungsmittel. verbleibt ein dünner Film des Beschichtungspolymers auf der Oberfläche der Vorrichtung, welcher die reversible Nukleinsäurebindung ermöglicht. Dem einschlägigen Fachmann sind solche Beschichtungsverfahren geläufig.

Die vorliegende Erfindung stellt weiterhin ein Verfahren zur Normalisierung von Nukleinsäure-Konzentrationen, bevorzugt zur Normalisierung von Nukleinsäure-Konzentrationen bei enzymatischen Nukleinsäure-Amplifikations- und Modifikations-Verfahren, zur Verfügung. Grundsätzlich umfasst das erfindungsgemäße Verfahren die Merkmale des Anspruchs 1.

Nukleinsäure enthaltende Proben im Sinne der Erfindung sind Lösungen, die bereits gereinigte Nukleinsäuren enthalten. Bei diesen Nukleinsäuren kann es sich um DNA oder RNA handeln, beispielsweise um Plasmide, PCR-Produkte oder eine Aufarbeitung von DNA, beispielsweise genomischer DNA, oder RNA oder von totaler Nukleinsäure aus einer biologischen Probe handeln. Gereinigt im Sinne der Erfindung sind Nukleinsäuren in Lösungen dann, wenn sie nicht in einem 'crude lysate' einer biologischen Probe vorliegen.

Die Inkubationszeit und die jeweiligen Bindebedingungen, um die Nukleinsäure an die modifizierte Oberfläche zu binden, sind abhängig von der jeweils verwendeten Art der Oberflächenmodifizierung, gehören aber zum Stand der Technik und sind für den Fachmann offensichtlich oder durch einfachste Routinearbeit bestimmbar.

Nachdem die Nukleinsäure an die modifizierte Oberfläche gebunden hat, kann der Rest der Probe verworfen bzw. die Probe von der Oberfläche oder die Oberfläche von der Probe entfernt werden werden. Liegt die erfindungsgemäße Vorrichtung beispielsweise in Form eines Gefäßes, z.B. Eppendorf-Tubes oder Multiwell-Platten, vor, kann die Probe einfach abgeschüttet oder abgesaugt bzw. abpipettiert werden. Liegt die erfindungsgemäße Vorrichtung beispielsweise in Form eines Dip-Sticks vor, kann dieser einfach aus der Probe entfernt werden.

Die Oberfläche, an die die Nukleinsäure aus der Probe nun gebunden ist, kann optional gewaschen werden. Hierzu wird eine Flüssigkeit verwendet, beispielsweise ein geeigneter Puffer, mit der Kontaminanten abgewaschen werden können, die Nukleinsäuren jedoch gebunden bleiben. Solche Flüssigkeiten gehören zum Stand der Technik und sind dem Fachmann daher wohlbekannt. Die zum Waschen verwendete Flüssigkeit wird anschließend verworfen.

Die Vorrichtung mit den gebundenen Nukleinsäuren kann im Anschluss einer nachfolgenden Reaktion, beispielsweise einer PCR oder RT-PCR, zugeführt werden. Für den Fall, dass die erfindungsgemäße Vorrichtung ein Gefäß, wie etwa Eppendorf-Tubes oder Multiwell-Platten, darstellt, wird die für die nachfolgende Reaktion benötigte Lösung in das Gefäß hinein gegeben. Ist z.B. die nachfolgende Reaktion eine PCR, so werden alle für die PCR notwendigen Komponenten (mit Ausnahme der zu amplifizierenden Nukleinsäure) in die erfindungsgemäße Vorrichtung in Form eines PCR-Gefäßes gegeben und die Reaktion wird gestartet. Durch das Erhitzen der Lösung zu Beginn der PCR lösen sich die Nukleinsäuren aus der reversiblen Bindung und die Reaktion kann mit einer definierten Menge Nukleinsäuren ablaufen. Für den Fall, dass die erfindungsgemäße Vorrichtung ein Dip-Stick oder eine äquivalente Vorrichtung ist, wird der Teil der Vorrichtung, an den die Nukleinsäuren gebunden sind, in eine entsprechende Vorrichtung gegeben, in der die nachfolgende Reaktion abläuft. Ist die nachfolgende Reaktion beispielsweise eine PCR, so wird der Dip-Stick oder der Teil des Dip-Sticks einfach in ein bereits alle Komponenten (mit Ausnahme der zu amplifizierenden Nukleinsäure) für die PCR enthaltendes PCR-Tube gegeben. Der weitere Ablauf ist wie oben beschrieben. Wird die erfindungsgemäße Vorrichtung in Form eines Dip-Sticks verwendet, so hat es sich als vorteilhaft erwiesen, dass die Dip-Sticks eine Sollbruchstelle aufweisen, mittels derer der die Nukleinsäuren bindende Teil leicht vom Rest der Vorrichtung getrennt werden kann. Dieser kann während der nachfolgenden Reaktion im Reaktionsgefäß verbleiben.

Verfahren, die eine Normierung der Nukleinsäuremengen voraussetzen oder bei denen sich eine solche als sehr vorteilhaft erweist, sind dem Fachmann hinlänglich bekannt und können dem erfindungsgemäßen Verfahren nachgeschaltet sein.

Alternativ kann die im erfindungsgemäßen Verfahren eingesetzte Vorrichtung auch eine zweite modifizierte Oberfläche mit definierter Nukleinsäure-Bindungskapazität aufweisen, wobei auf dieser modifizierten Oberfläche bereits als Standard dienende Nukleinsäuren immobilisiert sind. Diese Ausführungsform der Erfindung ist oben näher beschrieben. Hierbei ist, wie bereits oben erwähnt, von entscheidender Bedeutung, dass sich die als Standard dienenden Nukleinsäuren erst mit Beginn der nachfolgende Reaktion (z.B. PCR, RT-PCR, etc.) von dieser modifizierten Oberfläche lösen, z.B. durch das initiale Erhitzen der Lösung in einer PCR-Reaktion, und das diese zweite modifizierte Oberfläche vollständig gesättigt ist mit der als Standard dienenden Nukleinsäure, damit sie nicht für die in der Probe vorliegenden Nukleinsäure eine restliche Bindekapazität zeigt und so eine exakte Normierung nicht mehr möglich ist.

Die vorliegende Erfindung betrifft weiterhin Kits zur Durchführung des erfindungsgemäßen Verfahrens gemäβ Anspruch 4.

## Patentansprüche

1. Verfahren zur Normalisierung von Nukleinsäure-Konzentrationen aufweisend die folgenden Verfahrensschritte:
a. in Kontakt Bringen einer Nukleinsäure enthaltenden Probe mit einer Vorrichtung, aufweisend eine durch chemische Funktionalisierung modifizierte Oberfläche mit definierter Nukleinsäure-Bindekapazität zur reversiblen Bindung von Nukleinsäuren, wobei die modifizierte Oberfläche auf ionischen Schichten und/oder auf hydrophoben Schichten beruht,
b. Inkubieren unter Bedingungen und für einen Zeitraum, bis die Nukleinsäure-Bindekapazität der modifizierten Oberfläche dieser Vorrichtung erschöpft ist,
c. Verwerfen der restlichen Probe,
d. optional Waschen dieser Vorrichtung,
e. Zuführen dieser Vorrichtung einer nachfolgenden Reaktion.

2. Verfahren gemäß Anspruch 1, **dadurch gekennzeichnet, dass** die nachfolgende Reaktion aus Schritt e. ein enzymatisches Nukleinsäure-Amplifikations- oder Modifikations-Verfahren ist.

3. Verfahren gemäß Anspruch 2, **dadurch gekennzeichnet, dass** die nachfolgende Reaktion aus Schritt e. eine PCR oder RT-PCR ist.

4. Kit zur Durchführung eines Verfahrens gemäß einem der Ansprüche 1 bis 3 aufweisend eine Vorrichtung aufweisend eine durch chemische Funktionalisierung modifizierte Oberfläche mit definierter Nukleinsäure-Bindekapazität zur reversiblen Bindung von Nukleinsäuren, wobei die modifizierte Oberfläche auf ionischen Schichten und/oder auf hydrophoben Schichten beruht.

## Claims

1. Method for normalizing nucleic acid concentrations, comprising the following method steps:
a. contacting a nucleic acid-containing sample with a device comprising a surface which has been modified by chemical functionalization and has a defined nucleic acid-binding capacity for reversible binding of nucleic acids, wherein the modified surface is based on ionic layers and/or on hydrophobic layers,
b. incubating under such conditions and for such a period until the nucleic acid-binding capacity of the modified surface of said device is exhausted,
c. discarding the remainder of the sample,
d. optionally washing said device,
e. transferring said device to a subsequent reaction.

2. Method according to Claim 1, **characterized in that** the subsequent reaction in step e. is an enzymatic nucleic acid amplification or modification method.

3. Method according to Claim 2, **characterized in that** the subsequent reaction in step e. is a PCR or RT-PCR.

4. Kit for carrying out a method according to any of Claims 1 to 3, comprising a device comprising a surface which has been modified by chemical functionalization and has a defined nucleic acid-binding capacity for reversible binding of nucleic acids, wherein the modified surface is based on ionic layers and/or on hydrophobic layers.

## Revendications

1. Procédé pour la normalisation de concentrations en acide nucléique présentant les étapes de procédé suivantes :
a. la mise en contact d'un échantillon contenant des acides nucléiques avec un dispositif présentant une surface modifiée par une fonctionnalisation chimique, présentant une capacité de liaison d'acides nucléiques définie pour la liaison réversible d'acides nucléiques, la surface modifiée reposant sur des couches ioniques et/ou sur des couches hydrophobes,
b. l'incubation dans des conditions telles et pendant un laps de temps jusqu'à ce que la capacité de liaison de l'acide nucléique de la surface modifiée de ce dispositif soit épuisée,
c. rejet du reste de l'échantillon,
d. éventuellement lavage de ce dispositif,
e. introduction de ce dispositif dans une réaction consécutive.

2. Procédé selon la revendication 1, **caractérisé en ce que** la réaction consécutive de l'étape e. est un procédé d'amplification ou de modification enzymatique d'acides nucléiques.

3. Procédé selon la revendication 2, **caractérisé en ce que** la réaction consécutive de l'étape e. est une PCR ou une RT-PCR.

4. Kit pour la réalisation d'un procédé selon l'une quelconque des revendications 1 à 3, présentant un dispositif présentant une surface modifiée par une fonctionnalisation chimique, présentant une capacité de liaison d'acides nucléiques définie pour la liaison réversible d'acides nucléiques, la surface modifiée reposant sur des couches ioniques et/ou sur des couches hydrophobes,
